(19) **Europäisches Patentamt / European Patent Office / Office européen des brevets**

(11) **EP 2 378 962 B1**

(12) **FASCICULE DE BREVET EUROPEEN**

(45) Date de publication et mention de la délivrance du brevet:
**08.02.2017 Bulletin 2017/06**

(21) Numéro de dépôt: **09771388.7**

(22) Date de dépôt: **18.12.2009**

(51) Int Cl.:
**A61B 5/053** (2006.01)　　　**A61B 5/0402** (2006.01)

(86) Numéro de dépôt international:
**PCT/EP2009/067593**

(87) Numéro de publication internationale:
**WO 2010/070131 (24.06.2010 Gazette 2010/25)**

(54) **PROCEDE DE MESURE D'UN INDICE DE LA RIGIDITE LOCALE DE LA PAROI D'UNE ARTERE DE CONDUCTION ET INSTALLATION CORRESPONDANTE**

VERFAHREN ZUR MESSUNG EINES LOKALEN INDEX DER RIGIDITÄT DER WAND EINER LEITENDEN ARTERIE

METHOD OF MEASUREMENT OF A LOCAL RIGIDITY INDEX OF THE WALL OF A CONDUCTIVE ARTERY AND CORRESPONDING APPARATUS

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **19.12.2008 FR 0807264**

(43) Date de publication de la demande:
**26.10.2011 Bulletin 2011/43**

(73) Titulaires:
• **Association d'Enseignement Technique Supérieur du Groupe ESAIP**
**49124 Saint-Barthelemy D' Anjou (FR)**
• **Université d'Angers**
**49035 Angers Cedex (FR)**
• **CHU d'Angers**
**49933 Angers Cedex 9 (FR)**

(72) Inventeurs:
• **COLLETTE, Mathieu**
**49250 Beaufort en Vallée (FR)**
• **HUMEAU-HEURTIER, Anne**
**49170 Savennières (FR)**
• **LEFTHERIOTIS, Georges**
**49190 Rochefort Sur Loire (FR)**

(74) Mandataire: **Vidon Brevets & Stratégie**
**16B, rue de Jouanet**
**B.P. 90333**
**Technopole Atalante**
**35703 Rennes Cedex 7 (FR)**

(56) Documents cités:
FR-A- 2 707 860　　US-A- 4 030 485
US-A- 4 418 700　　US-A- 4 562 843
US-A1- 2007 016 083　　US-B1- 6 577 897

• LAURENT STEPHANE ET AL: "Expert consensus document on arterial stiffness: methodological issues and clinical applications" EUROPEAN HEART JOURNAL, vol. 27, no. 21, novembre 2006 (2006-11), pages 2588-2605, XP002541854 ISSN: 0195-668X
• RISACHER F: "IMPEDANCE PLETHYSMOGRAPHY FOR THE EVALUATION OF PULSE-WAVE VELOCITY IN LIMBS" MEDICAL AND BIOLOGICAL ENGINEERING AND COMPUTING, SPRINGER, HEILDELBERG, DE, vol. 31, no. 3, 1 mai 1993 (1993-05-01), pages 318-322, XP000367220 ISSN: 0140-0118
• COLLETTE MATHIEU ET AL: "Modeling and interpretation of the bioelectrical impedance signal for the determination of the local arterial stiffness" MEDICAL PHYSICS, AIP, MELVILLE, NY, US, vol. 36, no. 10, 4 septembre 2009 (2009-09-04), pages 4340-4348, XP012129705 ISSN: 0094-2405

## Description

### 1. Domaine de l'invention

Le domaine de l'invention est celui des techniques de détermination de la rigidité des artères de conduction de l'être humain ou de l'animal.

Plus précisément, l'invention concerne un procédé et une installation de détermination d'un indice de la rigidité locale de la paroi d'une artère de conduction véhiculant le sang d'un sujet.

### 2. Art antérieur

Les maladies cardiovasculaires demeurent actuellement la première cause de mortalité dans les pays développés. Ceci est notamment lié au fait qu'on y observe une augmentation constante des facteurs de risque cardio vasculaire dans les populations.

Un grand nombre d'études a montré une association forte entre le risque d'un accident cardiovasculaire et les altérations de la structure et/ou des fonctions pariétales vasculaires. La rigidification de l'arbre vasculaire est physiologiquement liée à l'âge et s'accélère avec le développement de la maladie athéromateuse favorisée par des facteurs de risque au rang desquels figurent le diabète, l'hypertension, la consommation de tabac, l'hypercholestérolémie, l'hérédité, la sédentarité...

L'artériosclérose reste longtemps asymptomatique au cours des premières décennies de la vie, se révélant plus tard par un symptôme ou un accident aigu, parfois fatal.

Il est donc nécessaire, afin de réduire les méfaits des maladies dégénératives du système vasculaire et en particulier l'artériosclérose, d'en améliorer le dépistage, afin de prévenir leur apparition, ou tout au moins d'anticiper ou de stopper leur développement par une prise en charge précoce des patients.

La sclérose de la paroi artérielle s'accompagne le plus souvent d'une augmentation de la rigidité de la paroi de l'artère.

A ce jour, différentes techniques peuvent être mises en oeuvre dans le but de déterminer la rigidité d'une artère de conduction.

Un indice de la rigidité régionale de la paroi artérielle aortique peut être obtenu en mesurant la vitesse de conduction de l'onde de pouls (en mètre par seconde) par tonométrie en deux points (carotide et fémoral). Cette technique, non traumatique, est actuellement considérée comme étant la référence. Toutefois, son utilisation en routine reste encore fastidieuse, délicate, et les résultats obtenus dépendent, en grande partie, de l'expertise de l'opérateur et de la morphologie du patient. De plus, cette technique permet seulement d'analyser l'aorte, principale artère élastique de l'organisme et ne permet d'obtenir qu'un indice régional et non un indice local de la rigidité de l'artère.

Un indice régional de la rigidité est un indice représentatif de la rigidité de l'ensemble d'une artère. Par opposition, un indice local de la rigidité est un indice représentatif de la rigidité d'une portion (ou d'un segment) d'une artère.

Des techniques ultrasonores (comme par exemple l'échographie) permettent également d'estimer la compliance (élasticité) vasculaire. La bonne mise en oeuvre de ces techniques dépend toutefois de l'expertise de l'opérateur, et reste strictement manuelle. En outre, bien qu'elles offrent des informations morphologiques utiles (visualisation de l'artère et de ses parois), elles ne peuvent pas être proposées dans le dépistage systématique et le diagnostic des maladies cardiovasculaires du fait de leur coût et de la durée de chaque examen.

Une autre technique consiste à étudier la morphologie des ondes de réflexion du signal de la pression artérielle enregistrée au doigt afin de déterminer la rigidité des artères. Cette technique permet seulement d'obtenir un indice de la rigidité régionale de l'arbre artériel et non un indice de la rigidité locale d'une artère.

La demande de brevet japonais portant le numéro JP2003169779 décrit une autre technique qui consiste à mesurer la vitesse de propagation d'une onde d'impédance véhiculée dans une artère de façon à estimer la vitesse de conduction de l'onde de pouls, et à en déduire un indice de la rigidité locale de la paroi aortique. Cette technique présente notamment l'inconvénient de nécessiter l'enregistrement successif du signal impédancemétrique sur deux sites anatomiques distincts afin de déterminer un indice de la rigidité locale de la paroi d'une artère.

LAURENT STEPHANE ET AL: "Expert consensus document on arterial stiffness: methodological issues and clinical applications", EUROPEAN HEART JOURNAL, vol. 27, no. 21, novembre 2006 (2006-11), pages 2588-2605, montre la mesure d'un indice local de rigidité de la paroi d'une artère. RISACHER F: "IMPEDANCE PLETHYSMOGRAPHY FOR THE EVALUATION OF PULSE-WAVE VELOCITY IN LIMBS", MEDICAL AND BIOLOGICAL ENGINEERING AND COMPUTING, SPRINGER, HEILDELBERG, DE, vol. 31, no. 3, 1 mai 1993 (1993-05-01), pages 318-322, montre la mesure de la variation d'impédance électrique d'un volume du sang. FR 2 707 860 A et US 4 562 843 A montrent la mesure de la variation d'impédance électrique d'un volume du sang. US 6 577 897 B1 montre la mesure de la variation d'impédance électrique d'un volume du sang et le calcul d'un indice d'élasticité.

### 3. Inconvénients de l'art antérieur

Ces techniques de l'art antérieur présentent donc pour la plupart les inconvénients suivants :

- elles sont relativement difficiles à mettre en oeuvre et requièrent un certain niveau d'expertise ;
- elles sont coûteuses à mettre en oeuvre.

Par ailleurs, on sait que la rigidification de la paroi d'une artère peut résulter de diverses caractéristiques.
La rigidification de la paroi d'une artère peut notamment résulter d'une caractéristique dite résistive qui traduit une augmentation de la pression intramurale liée à une augmentation de la résistance périphérique. La résistance périphérique est définie comme le rapport entre la pression différentielle (c'est-à-dire la pression systolique à laquelle on retranche la pression diastolique) et le débit artériel. La résistance périphérique s'oppose à l'écoulement du sang dans l'artère en phase systolique, ce qui engendre une augmentation de la pression différentielle à l'intérieur de l'artère. L'augmentation de la pression tend à entraîner une dilatation de l'artère. Dans le cas où une artère est dilatée au maximum, c'est-à-dire que son rayon ne peut plus être augmenté, l'artère apparaît comme rigide. La composante résistive est représentative de ce phénomène.
La rigidification de la paroi d'une artère peut encore résulter d'une diminution de sa caractéristique dite capacitive qui traduit la capacité d'une artère à emmagasiner de l'énergie mécanique due à la déformation de l'artère en phase systolique et à la restituer en phase diastolique.
Pourtant, les techniques de l'art antérieur conduisent seulement à obtenir un indice représentatif de la rigidité régionale ou locale d'une artère sans donner d'information sur les caractéristiques participant à cette rigidification ni sur leur importance.

### 4. Objectifs de l'invention

L'invention a notamment pour objectif de pallier ces inconvénients de l'art antérieur.
Plus précisément, un objectif de l'invention est de fournir une technique de détermination de la rigidité locale d'une artère de conduction véhiculant le sang d'un sujet.
L'invention vise notamment à fournir une telle technique qui permette de connaître l'influence d'au moins certaines caractéristiques mises en jeu dans la rigidification d'une artère.
L'invention vise également à fournir une telle technique qui soit fiable et précise.
Un autre objectif de l'invention est de produire une telle technique qui soit simple à mettre en oeuvre.
Un autre objectif de l'invention est d'effectuer la mesure sur un seul site anatomique.
L'invention a encore pour objectif de fournir une telle technique qui soit relativement peu coûteuse à mettre en oeuvre.

### 5. Exposé de l'invention

Ces objectifs, ainsi que d'autres qui apparaîtront par la suite, sont atteints à l'aide d'un procédé de mesure d'un indice (Ira) de la rigidité locale de la paroi d'une artère de conduction véhiculant le sang d'un sujet.
Selon l'invention, un tel procédé comprend au moins :

- une étape de mesure, en un unique point de mesure, de la variation d'impédance électrique ($\Delta Z$) d'un volume (V) du sang circulant dans un segment de ladite artère ;
- une étape de détermination d'un premier indice intermédiaire ($RP_\%$, RP) représentatif d'une caractéristique résistive mise en jeu dans la rigidification de ladite paroi, et d'un deuxième indice intermédiaire ($PCPA_\%$, ID) représentatif d'une caractéristique capacitive mise en jeu dans la rigidification de ladite paroi, lesdits premier ($RP_\%$, RP) et deuxième ($PCPA_\%$, ID) indices intermédiaires étant obtenus à partir de ladite mesure de la variation d'impédance électrique ($\Delta Z$) ;
- une étape de détermination dudit indice (Ira) de la rigidité locale en fonction desdits premier ($RP_\%$, RP) et deuxième ($PCPA_\%$, ID) indices intermédiaires.

Ainsi, l'invention repose sur une approche tout à fait nouvelle et inventive qui consiste à déterminer au moins deux indices intermédiaires chacun représentatif d'une caractéristique résistive et d'une caractéristique capacitive entrant dans la rigidification d'une artère, puis à déterminer un indice global de la rigidité locale de la paroi d'une artère en fonction des indices intermédiaires précédemment déterminés.
Les inventeurs ont découvert que la rigidification de la paroi d'une artère peut notamment résulter d'une caractéristique dite résistive et d'une caractéristique dite capacitive. La définition de la rigidité locale de la paroi d'une artère suppose donc d'évaluer les caractéristiques résistive et capacitive mises en jeu dans la rigidification globale de la paroi de l'artère. La connaissance de chacune de ces caractéristiques résistive et capacitive permet de déterminer un indice de la rigidité locale de la paroi d'une artère qui soit particulièrement précis et représentatif de la réalité.

La caractéristique résistive traduit une augmentation du rapport entre la pression intramurale et le débit artériel. Il peut par exemple s'agir de la résistance périphérique ou locale.

La caractéristique capacitive traduit la capacité d'une artère à emmagasiner de l'énergie mécanique due à la déformation de l'artère en phase systolique et à la restituer en phase diastolique. Elle est donc liée à l'élasticité de l'artère. Il peut par exemple s'agir de la distensibilité de l'artère.

Ainsi, la mise en oeuvre de l'invention permet :

- d'obtenir un indice global qui permet de connaître de manière précise et réaliste le niveau de la rigidité locale de la paroi d'une artère dont la prise en compte permet de savoir si l'artère est en un point plutôt rigide ou plutôt souple, et
- d'obtenir deux indices intermédiaires dont la prise en compte permet de connaître l'importance respective d'une caractéristique résistive et d'une caractéristique capacitive de l'artère entrant dans sa rigidification locale.

La mise en oeuvre de l'invention permet donc à la personne en charge de l'analyse des résultats obtenus d'avoir une image plus précise de la rigidité d'un segment d'une artère et notamment de connaître l'importance des caractéristiques diverses qui en sont à l'origine. Ces connaissances peuvent par la suite permettre de soigner de manière plus efficace un patient en lui administrant, par exemple, un traitement ciblé pour chacune des caractéristiques entrant dans la rigidification de ses artères.

En outre, la mise en oeuvre de l'invention ne nécessite que la mesure d'une variation d'impédance dans un volume de sang circulant dans un segment d'une artère, et ne nécessite pas, comme cela est le cas selon l'art antérieur, d'effectuer successivement deux mesures de la variation d'impédance sur deux sites anatomiques distincts. La présente invention est donc relativement simple à mettre en oeuvre.

Selon un premier mode de réalisation avantageux, ledit premier indice intermédiaire (RP%) est un indice représentatif de la résistance périphérique en aval dudit segment au cours d'une phase systolique d'un battement cardiaque, et ledit deuxième indice intermédiaire (PCPA%) est indice représentatif de la capacité de ladite artère à emmagasiner une énergie mécanique due à la déformation de ladite artère durant ladite phase systolique dudit battement cardiaque et à la restituer durant la phase diastolique dudit battement cardiaque.

La mise en oeuvre de l'invention permet donc d'obtenir des indices représentatifs des caractéristiques dite résistive et dite capacitive participant à la rigidification de l'artère et sur leur importance dans cette rigidification.

Préférentiellement, ladite étape de détermination dudit indice (Ira) de la rigidité locale comprend une étape de calcul selon la formule :

$$Ira = (1 - |PCPA_\%|) \times RP_\% + (1 - RP_\%) * |PCPA_\%|$$

Cette formule permet, à partir des deux indices intermédiaires, de déterminer un indice représentatif de la rigidité locale de la paroi d'un segment d'artère de manière efficace et précise.

Un procédé selon l'invention comprend avantageusement une étape de calcul dudit deuxième indice intermédiaire (PCPA%) selon la formule :

$$PCPA_\% = \frac{J - I}{J + I} * 100$$

$$\text{avec} \quad I = \int_{t_1}^{t_2} \left| \frac{d}{dt} \left( \frac{1}{\Delta Z} \right) \right| dt \text{ et } J = \int_{t_2}^{t_3} \left| \frac{d}{dt} \left( \frac{1}{\Delta Z} \right) \right| dt,$$

$t_1$ représentant le temps d'apparition du pied de la dérivée de la variation d'impédance ($\frac{d}{dt} \frac{1}{\Delta Z}$) depuis le début de ladite phase systolique,

$t_2$ représentant le temps d'apparition du maximum de la dérivée de la variation d'impédance ($\frac{d}{dt} \frac{1}{\Delta Z}$) depuis le début de ladite phase systolique,

$t_3$ représentant le temps d'apparition de l'intersection de la dérivée de la variation d'impédance $(\frac{d}{dt}\frac{1}{\Delta Z})$ et d'une

droite parallèle à l'axe des abscisses passant par le point de la courbe $(\frac{d}{dt}\frac{1}{\Delta Z})$ au temps $t_1$.

Cette formule permet, à partir de la mesure de variation d'impédance, de déterminer un indice représentatif de la caractéristique capacitive de l'artère de manière efficace et précise.

Selon une autre caractéristique avantageuse, un procédé selon l'invention comprend une étape de calcul dudit premier indice intermédiaire (RP$_{\%}$) selon la formule :

$$RP_{\%} = \frac{K-I}{K} * 100$$

K étant une constante dépendant des moyens mis en oeuvre pour réaliser ladite étape de mesure de la variation d'impédance électrique ($\Delta Z$).

Cette formule permet, à partir de la mesure de variation d'impédance, de déterminer un indice représentatif de la caractéristique résistive de l'artère de manière efficace et précise.

Selon un deuxième mode de réalisation avantageux, ledit premier indice intermédiaire est un indice (RP) représentatif de la résistance locale dudit segment au cours d'une phase systolique d'un battement cardiaque, et ledit deuxième indice intermédiaire est un indice (ID) représentatif de la distensibilité de ladite artère au cours d'une phase systolique d'un battement cardiaque.

La mise en oeuvre de l'invention permet donc d'obtenir des indices représentatifs des caractéristiques dite résistive et dite capacitive participant à la rigidification de l'artère et sur leur importance dans cette rigidification.

Dans ce cas, un procédé selon l'invention comprend préférentiellement une étape de mesure de la pression artérielle en phase systolique (PAS), de la pression artérielle en phase diastolique (PAD) et de calcul de la pression artérielle moyenne (PAM).

Ladite étape de détermination dudit indice (Ira) de la rigidité locale comprend avantageusement une étape de calcul selon la formule :

$$Ira = \frac{PAS - PAD}{PAM} \cdot \frac{RP \cdot ID}{RP + ID}$$

Cette formule permet, à partir des deux indices intermédiaires, de déterminer un indice représentatif de la rigidité locale de la paroi d'un segment d'artère de manière efficace et précise.

Préférentiellement, un procédé selon l'invention comprend une étape de calcul dudit premier indice intermédiaire (RP) selon la formule

$$RP = \frac{PAM}{(I + J)}$$

$$\text{avec } I = \int_{t_1}^{t_2} \left| \frac{d}{dt}\left(\frac{1}{\Delta Z}\right) \right| dt \text{ et } J = \int_{t_2}^{t_3} \left| \frac{d}{dt}\left(\frac{1}{\Delta Z}\right) \right| dt,$$

$t_1$ représentant le temps d'apparition du pied de la dérivée de la variation d'impédance $(\frac{d}{dt}\frac{1}{\Delta Z})$ depuis le début de ladite phase systolique,

$t_2$ représentant le temps d'apparition du maximum de la dérivée de la variation d'impédance $(\frac{d}{dt}\frac{1}{\Delta Z})$ depuis le début de ladite phase systolique,

$t_3$ représentant le temps d'apparition de l'intersection de la dérivée de la variation d'impédance ($\frac{d}{dt}\frac{1}{\Delta Z}$) et d'une

droite parallèle à l'axe des abscisses passant par le point de la courbe ($\frac{d}{dt}\frac{1}{\Delta Z}$) au temps $t_1$.

Cette formule permet, à partir de la mesure de variation d'impédance, de déterminer un indice représentatif de la caractéristique résistive de l'artère de manière efficace et précise.

Préférentiellement, un procédé selon l'invention comprend une étape de calcul dudit deuxième indice intermédiaire (ID) selon la formule :

$$ID = PAM\frac{100\big[2(J-I)-(J+I)\big]}{(J+I)^2}$$

Cette formule permet, à partir de la mesure de variation d'impédance, de déterminer un indice représentatif de la caractéristique capacitive de l'artère de manière efficace et précise.

Selon un mode de réalisation particulier, un procédé selon l'invention comprend une étape d'acquisition du signal électrocardiogramme (ECG) dudit sujet, et une étape de synchronisation dudit signal électrocardiogramme (ECG) et de ladite variation d'impédance ($\Delta Z$).

De cette manière, les indices Ira, $RP_\%$, RP et $PCPA_\%$, ID peuvent être calculés pour chaque battement cardiaque.

Préférentiellement, un procédé selon l'invention comprend plusieurs :

- étapes de détermination dudit premier ($RP_\%$, RP) et dudit deuxième ($PCPA_\%$, ID) indices intermédiaires ;
- étapes de détermination dudit indice (Ira) de la rigidité locale en fonction desdits premier ($RP_\%$, RP) et deuxième ($PCPA_\%$, ID) indices intermédiaires,

lesdites étapes de détermination étant menées au cours de battements cardiaques (R) successifs, ledit procédé comprenant de plus une étape de calcul de la moyenne de chacun desdits indices (Ira, $RP_\%$, RP, $PCPA_\%$, ID) au cours desdits battements (R).

Cette mise en oeuvre particulière permet d'améliorer la précision des résultats obtenus.

Avantageusement, un procédé selon l'invention comprend plusieurs étapes de mesure, en un unique point de mesure, de la variation d'impédance électrique ($\Delta Z$) d'un volume (V) du sang circulant dans un segment de ladite artère, chacune desdites mesures étant réalisées sur des battements cardiaques (R) différents, ledit procédé comprenant de plus une étape de détermination de la moyenne de la variation d'impédance sur lesdits battement cardiaques et une étape de détermination dudit premier ($RP_\%$, RP) et dudit deuxième ($PCPA_\%$, ID) en fonction de ladite moyenne.

Cette mise en oeuvre permet également d'améliorer la précision des résultats obtenus.

Selon une caractéristique préférentielle, un procédé selon l'invention comprend une étape d'affichage dudit indice (Ira) de la rigidité locale de la paroi d'une artère de conduction, et une étape d'affichage desdits premier ($RP_\%$, RP) et deuxième ($PCPA_\%$, ID) indices intermédiaires.

Les résultats de la mesure peuvent ainsi être directement exploités par un praticien de manière à l'aider à diagnostiquer l'état clinique d'un patient par exemple en vue de lui administrer un traitement adapté.

L'invention porte également sur une installation pour la mise en oeuvre du procédé de détermination d'un indice (Ira) de la rigidité locale de la paroi d'une artère de conduction d'un sujet véhiculant du sang.

Selon l'invention, une telle installation comprend :

- des moyens de mesure, en un unique point de mesure, de la variation d'impédance électrique ($\Delta Z$) d'un volume (V) du sang circulant dans un segment ladite artère ;
- des moyens de détermination d'un premier indice intermédiaire ($RP_\%$, RP) représentatif d'une caractéristique résistive mise en jeu dans la rigidification de ladite paroi, et d'un deuxième indice intermédiaire ($PCPA_\%$, ID) représentatif d'une caractéristique capacitive mise en jeu dans la rigidification de ladite paroi ; - des moyens de détermination dudit indice (Ira) de la rigidité locale en fonction desdits premier ($RP_\%$, RP) et deuxième ($PCPA_\%$, ID) indices intermédiaires.

Selon un premier mode de réalisation avantageux, lesdits moyens de détermination dudit premier indice intermédiaire ($RP_\%$) comprennent des moyens de détermination d'un indice représentatif de la résistance périphérique en aval dudit segment au cours d'une phase systolique d'un battement cardiaque, et lesdits moyens de détermination dudit deuxième indice intermédiaire (PCPA%) comprennent des moyens de détermination d'un indice représentatif de la capacité de ladite artère à emmagasiner une énergie mécanique due à la déformation de ladite artère durant ladite phase systolique dudit battement cardiaque et à la restituer durant la phase diastolique dudit battement cardiaque.

Dans ce cas, une installation selon l'invention comprend préférentiellement des moyens de calcul dudit indice (Ira) de la rigidité locale selon la formule :

$$Ira = (1 - \left|PCPA_{\%}\right|) \times RP_{\%} + (1 - RP_{\%}) * \left|PCPA_{\%}\right|$$

Selon une autre caractéristique avantageuse, lesdits moyens de calcul dudit deuxième indice intermédiaire (PCPA%) comprennent des moyens de calcul selon la formule :

$$PCPA_{\%} = \frac{J - I}{J + I} * 100$$

$$\text{avec } I = \int_{t_1}^{t_2} \left|\frac{d}{dt}\left(\frac{1}{\Delta Z}\right)\right| dt \text{ et } J = \int_{t_2}^{t_3} \left|\frac{d}{dt}\left(\frac{1}{\Delta Z}\right)\right| dt,$$

$t_1$ représentant le temps d'apparition du pied de la dérivée de la variation d'impédance $(\frac{d}{dt}\frac{1}{\Delta Z})$ depuis le début de ladite phase systolique,

$t_2$ représentant le temps d'apparition du maximum de la dérivée de la variation d'impédance $(\frac{d}{dt}\frac{1}{\Delta Z})$ depuis le début de ladite phase systolique,

$t_3$ représentant le temps d'apparition de l'intersection de la dérivée de la variation d'impédance $(\frac{d}{dt}\frac{1}{\Delta Z})$ et d'une droite parallèle à l'axe des abscisses passant par le point de la courbe $(\frac{d}{dt}\frac{1}{\Delta Z})$ au temps $t_1$.

Lesdits moyens de calcul dudit premier indice intermédiaire (RP%) comprend préférentiellement des moyens de calcul selon la formule :

$$RP_{\%} = \frac{K - I}{K} * 100$$

K étant une constante dépendant des moyens mis en oeuvre pour réaliser ladite étape de mesure de la variation d'impédance électrique ($\Delta Z$).

Selon un deuxième mode de réalisation avantageux, lesdits moyens de détermination dudit premier indice intermédiaire (RP) comprennent des moyens de détermination d'un indice représentatif de la résistance locale dudit segment au cours d'une phase systolique d'un battement cardiaque, et lesdits moyens de détermination dudit deuxième indice intermédiaire (ID) comprennent des moyens de détermination d'un indice représentatif de la distensibilité au cours d'une phase systolique d'un battement cardiaque.

Dans ce cas, une installation selon l'invention comprend préférentiellement des moyens de calculs dudit indice (Ira) de la rigidité locale selon la formule :

$$Ira = \frac{PAS - PAD}{PAM} \cdot \frac{RP \cdot ID}{RP + ID}$$

Lesdits moyens de calcul dudit premier indice intermédiaire (RP) comprennent préférentiellement des moyens de calcul selon la formule :

$$RP = \frac{PAM}{(I + J)}$$

$$\text{avec } I = \int_{t_1}^{t_2} \left| \frac{d}{dt}\left(\frac{1}{\Delta Z}\right) \right| dt \text{ et } J = \int_{t_2}^{t_3} \left| \frac{d}{dt}\left(\frac{1}{\Delta Z}\right) \right| dt,$$

$t_1$ représentant le temps d'apparition du pied de la dérivée de la variation d'impédance $\left(\dfrac{d}{dt}\dfrac{1}{\Delta Z}\right)$ depuis le début de ladite phase systolique,

$t_2$ représentant le temps d'apparition du maximum de la dérivée de la variation d'impédance $\left(\dfrac{d}{dt}\dfrac{1}{\Delta Z}\right)$ depuis le début de ladite phase systolique,

$t_3$ représentant le temps d'apparition de l'intersection de la dérivée de la variation d'impédance $\left(\dfrac{d}{dt}\dfrac{1}{\Delta Z}\right)$ et d'une droite parallèle à l'axe des abscisses passant par le point de la courbe $\left(\dfrac{d}{dt}\dfrac{1}{\Delta Z}\right)$ au temps $t_1$.

Lesdits moyens de calcul dudit deuxième indice intermédiaire (ID) comprennent préférentiellement des moyens de calcul selon la formule :

$$ID = PAM\,\frac{100\big[2(J-I)-(J+I)\big]}{(J+I)^2}$$

Une installation selon l'invention comprend avantageusement des moyens d'acquisition du signal d'électrocardiogramme (ECG) dudit sujet, des moyens de détection de chacun des battements cardiaques (R) apparaissant sur ledit électrocardiogramme (ECG), et des moyens d'activation desdits moyens de détermination desdits indices consécutivement à la détection d'au moins un battement cardiaque (R).

Chaque indice peut être déterminé selon l'invention pour un battement cardiaque. Dans une variante, chacun des indices peut être déterminé sur des battements cardiaques successifs, la valeur de l'indice obtenu au final correspondant à la moyenne des valeurs des indices déterminés successivement. Ceci permet d'augmenter la précision des résultats obtenus. Selon encore une autre variante, la variation d'impédance peut être mesurée successivement sur des battements cardiaques successifs. Une courbe correspondant à la moyenne de la variation d'impédance sur les différents battements peut ensuite être obtenue. Les différents indices peuvent alors être déterminés à partir de cette courbe moyenne. Cette mise en oeuvre permet également d'améliorer la précision des résultats.

## 6. Liste des figures

D'autres caractéristiques et avantages de l'invention apparaîtront plus clairement à la lecture de la description suivante d'un mode de réalisation préférentiel, donné à titre de simple exemple illustratif et non limitatif, et des dessins annexés, parmi lesquels :

- la figure 1 présente de façon schématique une installation pour la mise en oeuvre d'un procédé selon l'invention dans laquelle les électrodes sont positionnées de manière à déterminer la rigidité des parois de l'aorte ;
- la figure 2 illustre un positionnement des électrodes pour déterminer la rigidité de l'artère fémorale ;
- la figure 3a est une courbe représentant l'électrocardiogramme (ECG) d'un sujet ;
- la figure 3b est une courbe illustrant l'inverse de la variation d'impédance dans une volume (V) de sang circulant dans une portion d'artère placée entre les électrodes émettrices et réceptrices d'une installation selon l'invention ;
- la figure 3c est une courbe illustrant la dérivée de la courbe illustrée à la figure 3b ;
- la figure 4 illustre un organigramme d'un procédé selon l'invention.

## 7. Description d'un mode de réalisation de l'invention

### 7.1. Principe général de l'invention

Le principe général de l'invention repose sur le fait de déterminer deux indices intermédiaires respectivement représentatifs d'une caractéristique résistive et d'une caractéristique capacitive entrant dans la rigidification d'une artère, puis de déterminer un indice de la rigidité locale de la paroi d'une artère en fonction des deux indices intermédiaires précédemment déterminés.

Les inventeurs ont découvert que la rigidification de la paroi d'une artère peut notamment résulter d'une caractéristique dite résistive, liée à la résistance locale ou périphérique, de l'artère et d'une caractéristique dite capacitive liée à l'élasticité de l'artère. La connaissance de chacune de ces caractéristiques résistive et capacitive d'une artère permet de déterminer un indice de la rigidité locale de cette artère particulièrement précis et représentatif de la réalité.

La mise en oeuvre de l'invention permet donc d'une part d'obtenir un indice global permettant de connaître le niveau de la rigidité locale de la paroi d'une artère dont la prise en compte permet de savoir si l'artère est, le long d'un segment, plutôt rigide ou plutôt souple, et d'obtenir deux indices intermédiaires dont la prise en compte permet de connaître l'importance respective d'une caractéristique résistive et d'une caractéristique capacitive de l'artère entrant dans sa rigidification locale.

La prise en compte des résultats ainsi obtenus permet d'avoir une image plus précise de la rigidité d'une artère et notamment de connaître l'importance des caractéristiques diverses qui en sont à l'origine, et de soigner de manière plus efficace un patient en lui administrant, par exemple, un traitement ciblé pour chacune des caractéristiques entrant dans la rigidification de ses artères.

Par ailleurs, ces deux indices intermédiaires sont obtenus, à partir de la mesure, en un unique point, de la variation d'impédance électrique d'un volume de sang circulant dans un segment de l'artère dont on souhaite déterminer la rigidité. La mise en oeuvre de l'invention est ainsi facilitée.

### 7.2. Exemple d'un premier mode de réalisation d'une installation pour la mise en oeuvre d'un procédé selon l'invention

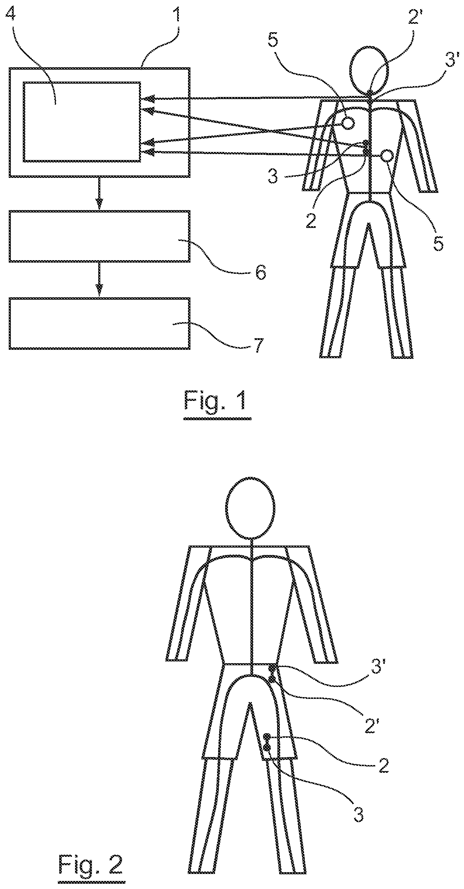

On présente, en relation avec la figure 1, un mode de réalisation d'une installation pour la mise en oeuvre d'un procédé selon l'invention.

Ainsi que cela est représenté sur cette figure 1, une telle installation comprend deux couples d'électrodes 2, 3 et 2', 3'. Chacun de ces couples d'électrodes comprend une électrode émettrice 2 ou 2' et une électrode réceptrice 3 ou 3'. Ces couples d'électrodes sont destinés à être positionnés sur un sujet de telle sorte qu'ils définissent un volume à l'intérieur duquel se trouve une artère dont il est souhaité de déterminer la rigidité, ces électrodes définissant un axe parallèle à l'axe principal de cette artère.

Une telle installation comprend également deux autres électrodes 5 destinées à permettre d'acquérir le signal électrocardiogramme du sujet.

Les électrodes 2, 3, 2', 3' sont reliées à un impédancemètre 1 du type de ceux qui se trouvent classiquement dans le commerce.

Un tel impédancemètre 1 comprend des moyens de synchronisation 4 permettant de synchroniser, avec le signal électrocardiogramme, un signal d'impédance mesuré dans un volume (V) de sang circulant à l'intérieur de la portion (ou du segment) de l'artère située entre les couples d'électrodes.

Cette installation comprend en outre des moyens de calculs 6, par exemple un ordinateur, qui sont reliés à l'impédancemètre 1 en vue de traiter les signaux qu'il délivre et de calculer un indice (Ira) représentatif de la rigidité locale de la paroi de l'artère étudiée de la manière qui sera décrite plus bas.

Elle comprend encore des moyens d'affichage 7 des résultats obtenus.

### 7.3. Exemple d'un premier mode de réalisation procédé selon l'invention

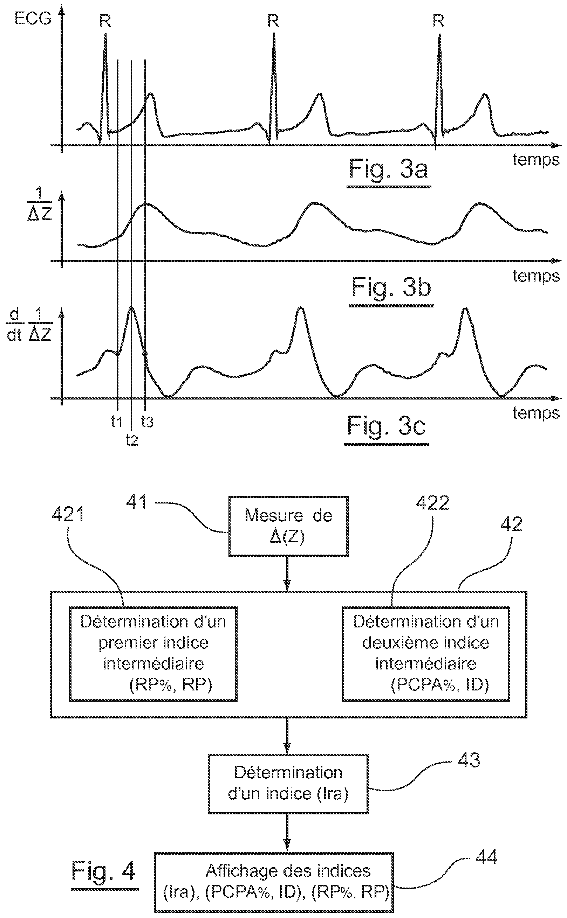

Un procédé de mesure d'un indice (Ira) de la rigidité locale de la paroi d'une artère de conduction véhiculant le sang d'un sujet va maintenant être décrit notamment en référence à la figure 4.

Un tel procédé consiste à positionner deux couples d'électrodes émettrices et d'électrodes réceptrices 2, 3 et 2', 3' sur un sujet de telle sorte qu'ils forment un axe parallèle à l'axe de l'artère dont on souhaite mesurer la rigidité et définissant un volume à l'intérieur duquel se trouve cette artère.

La figure 1 indique l'emplacement des électrodes 2, 3, 2', 3' au niveau du thorax pour l'étude de l'aorte. Les électrodes 2' et 3' sont positionnées à la base du cou, du même côté, l'une au dessus de l'autre, sans chevauchement et les électrodes 2 et 3 sont positionnées en dessous du sternum, l'une au dessus de l'autre, sans chevauchement.

La figure 2 illustre un exemple d'emplacement de ces électrodes au niveau de la cuisse pour l'étude de l'artère fémorale. Le positionnement de ces électrodes pourra bien sur être modifié de manière à couvrir d'autres zones anatomiques susceptibles de contenir une artère de conduction dont il est souhaité d'étudier la rigidité.

Par ailleurs, la présente invention peut aussi bien être mise en oeuvre chez l'homme ou chez l'animal, pourvu que le signal acquis soit représentatif de l'écoulement du sang dans l'artère de conduction étudiée.

Des électrodes 5 d'électrocardiogrammes sont également mises en place, par exemple, sur le thorax du sujet.

Les électrodes 2, 3, 2', 3', et 5 sont toutes reliées à un impédancemètre 1 qui permet :

- l'injection d'un courant électrique de faible intensité (de l'ordre de 3 mA) et de fréquence élevée réglable (de l'ordre de 75 kHz) à travers le volume (V) de sang circulant dans un segment d'artère placé entre les électrodes émettrices 2, 2' et les électrodes réceptrices 3, 3' ;
- la mesure (41) de la variation d'impédance ($\Delta Z$) dans ce volume de sang et l'acquisition d'un signal représentant l'inverse de la variation d'impédance ($\Delta Z$) (figure 3b) ;
- l'acquisition d'un signal d'électrocardiogramme (ECG) du sujet (figure 3a).

Il est noté que la mise en oeuvre de l'impédancemètre 1 ne s'accompagne d'aucune contrainte désagréable pour le sujet (pas de compression, pas de limitation de mouvements) et ne présente aucun risque d'utilisation puisque la technique est non invasive.

Les signaux délivrés par l'impédancemètre 1 sont transmis à des moyens de calculs, comme l'ordinateur 6, en vue :

- de déterminer (42) un premier indice intermédiaire (RP%) et un deuxième indice intermédiaire (PCPA%) respectivement représentatif d'une caractéristique résistive et d'une caractéristique capacitive mises en jeu dans la rigidification de la paroi du segment de l'artère étudiée ;
- de déterminer (43), en fonction desdits premier (RP%) et deuxième (PCPA%) indices intermédiaires, un indice (Ira) représentatif de la rigidité locale de la paroi de l'artère.

Dans ce mode de réalisation, l'ordinateur 6 permet, en fonction des signaux délivrés par l'impédancemètre 1 :

- de déterminer (421) le premier indice intermédiaire (RP%) qui est représentatif de la résistance périphérique en aval du segment artériel au cours d'une phase systolique d'un battement cardiaque ;
- de déterminer (422) le deuxième indice intermédiaire (PCPA%) qui représentatif de la capacité de l'artère à emmagasiner de l'énergie mécanique due à la déformation de l'artère durant la phase systolique du battement cardiaque et à la restituer durant la phase diastolique du battement cardiaque.

Plus précisément, l'ordinateur 6 traite trois signaux en vue de déterminer les indices (Ira), (RP%) et (PCPA%) :

- le signal d'électrocardiogramme (ECG) du sujet (figure 3a) ;
- le signal représentant l'inverse de la variation d'impédance dans le volume (V) de sang circulant dans la portion d'artère située entre les couples d'électrodes (figure 3b) ;
- le signal représentant la dérivée de l'inverse de la variation d'impédance (figure 3c).

Les moyens de calculs 6 déterminent l'indice intermédiaire (PCPA%) selon la formule :

$$PCPA_{\%} = \frac{J - I}{J + I} * 100$$

avec

$$I = \int_{t_1}^{t_2} \left| \frac{d}{dt}\left(\frac{1}{\Delta Z}\right) \right| dt$$

$$J = \int_{t_2}^{t_3} \left| \frac{d}{dt}\left(\frac{1}{\Delta Z}\right) \right| dt$$

et avec :

$t_1$ représentant le temps d'apparition du pied de la dérivée de la variation d'impédance ($\frac{d}{dt}\frac{1}{\Delta Z}$) depuis le début de la phase systolique,

$t_2$ représentant le temps d'apparition du maximum de la dérivée de la variation d'impédance ($\frac{d}{dt}\frac{1}{\Delta Z}$) depuis le début de la phase systolique,

$t_3$ représentant le temps d'apparition de l'intersection de la dérivée de la variation d'impédance ($\frac{d}{dt}\frac{1}{\Delta Z}$) et d'une droite parallèle à l'axe des abscisses passant par le point de la courbe ($\frac{d}{dt}\frac{1}{\Delta Z}$) au temps $t_1$.

Les moyens de calculs 6 déterminent l'indice intermédiaire (RP%) selon la formule :

$$RP_{\%} = \frac{K-I}{K}*100$$

K étant une constante dépendant des moyens mis en oeuvre pour réaliser la mesure de la variation d'impédance électrique ($\Delta Z$).

La constante K est obtenue en réalisant plusieurs mesures de la rigidité de la paroi d'une artère sur différents sujets :

-   par la mise en oeuvre d'une technique de l'art antérieur prise comme référence, et
-   par la mise en oeuvre de la technique selon l'invention.

La valeur de la constante K est alors ajustée de telle manière que la technique selon l'invention conduise à obtenir des valeurs de rigidité équivalentes aux valeurs obtenues selon l'art antérieur. À titre indicatif, cette constante K pourra être égale à 5000.

Les moyens de calculs 6 déterminent enfin l'indice (Ira) en fonction des indices intermédiaires (PCPA%) et (RP%) calculés précédemment selon la formule :

$$Ira = (1 - |PCPA_{\%}|) \times RP_{\%} + (1 - RP_{\%}) * |PCPA_{\%}|$$

La valeur des indices (Ira), (PCPA%) et (RP%) peut ensuite être affichée (44) sur les moyens d'affichage 7 en vue de pouvoir être analysée.

Dans une variante, ces moyens d'affichage 7 pourront être intégrés à une plate-forme virtuelle et les valeurs pourront être transmises à distance par les moyens de calcul ou directement par l'impédancemètre de manière telle que les résultats puissent être analysés en un lieu distant de celui dans lequel les mesures sont réalisées.

Les paramètres (Ira), (PCPA%) et RP%) sont calculés pour un battement cardiaque. L'impédancemètre 1 comprend donc des moyens de synchronisation 4 permettant de synchroniser le signal électrocardiogramme (ECG) et le signal d'impédance mesurée dans le volume (V) de sang circulant à l'intérieur de la portion de l'artère située entre les couples d'électrodes.

L'impédancemètre 1 ou les moyens de calcul 6 comprennent également des moyens qui permettent, par analyse du signal électrocardiogramme, de détecter la survenue d'un battement cardiaque (R) et de déclencher en conséquence l'activation des moyens de calculs 6 en vue d'obtenir la valeur des indices (Ira), (PCPA%) et (RP%).

Dans une variante de ce mode de réalisation, les indices (Ira), (PCPA%) et (RP%) peuvent correspondre à la moyenne des courbes réalisées sur des battements cardiaques (R) successifs. Ceci peut permettre d'améliorer la précision des résultats.

Il a été constaté que l'indice (Ira) calculé au niveau de l'aorte était fortement corrélé à la vitesse de propagation de l'onde de pouls mesurée par techniques tonométriques, dites de référence.

La mise en oeuvre de la présente invention permet de fournir non seulement un indice (Ira) de la rigidité locale de la paroi d'un segment d'une artère mais aussi deux autres indices intermédiaires (RP%) et (PCPA%) pouvant aider au diagnostic dans le but de préciser les caractéristiques responsables d'une rigidification de la paroi artérielle, de manière non invasive, simple, rapide, directe, sans manipulation de l'opérateur et pouvant s'appliquer sur l'ensemble des artères de conduction.

L'indice de la rigidité locale de la paroi aortique est fourni par l'indice (Ira) et l'importance relative de la composante résistive et de la composante capacitive responsables de la rigidité artérielle pourra être quantifiée et appréciée de l'homme de métier par l'interprétation des indices intermédiaires (RP$_\%$) et (PCPA$_\%$).

Il a ainsi été constaté expérimentalement, que les valeurs des indices intermédiaires (RP$_\%$) et (PCPA$_\%$) pourront être répertoriés en différentes classes, mentionnées ici à titre purement indicatif, illustrant l'état des artères :

- (RP$_\%$) inférieur à 50 % et (PCPA$_\%$) supérieur à 5 % indiquent une résistance périphérique basse et une élasticité aortique élevée.
- (RP$_\%$) inférieur à 50 % et (PCPA$_\%$) inférieur à -5 % indiquent une résistance périphérique basse et une élasticité aortique basse.
- (RP$_\%$) supérieur à 50 % et (PCPA$_\%$) inférieur à -5 % indiquent une résistance périphérique élevée et une élasticité aortique basse.
- (RP$_\%$) supérieur à 50 % et (PCPA$_\%$) supérieur à 5 % indiquent une résistance périphérique élevée et une élasticité aortique élevée.

La prise en compte des résultats ainsi obtenus permet d'avoir une image précise de la rigidité globale d'un segment d'une artère et de connaître l'importance des caractéristiques diverses qui en sont à l'origine. Un patient peut alors être soigné de manière plus efficace en lui administrant un traitement ciblé pour chacune des caractéristiques entrant dans la rigidification de ses artères.

La mise en oeuvre de la technique selon l'invention permet de déterminer, à un instant donné, la rigidité d'une artère de conduction quelconque. Elle peut également permettre d'en suivre l'évolution par des mesures successives. Elle peut ainsi permettre d'évaluer l'incidence par exemple de l'administration d'un traitement médicamenteux, de la réalisation d'un traitement physique interne ou externe ou de toute autre intervention sur la rigidité d'une artère d'un sujet.

L'invention peut également être mise en oeuvre comme test de type « screening » pour la mise au point de nouvelles molécules susceptibles de devenir des médicaments.

Elle peut aussi bien être mise en oeuvre chez l'homme que chez l'animal.

**7.4. Exemple d'un deuxième mode de réalisation d'une installation pour la mise en oeuvre d'un procédé selon l'invention**

Une installation selon le deuxième mode de réalisation se distingue de celle selon le premier mode de réalisation du fait qu'elle comprend en outre des moyens de mesure de la pression artérielle en phase systolique (PAS), des moyens de mesure de la pression artérielle en phase diastolique (PAD) et des moyens de calcul de la pression artérielle moyenne (PAM). Les moyens de détermination des indices, qui comprennent l'ordinateur 6, sont programmés pour exécuter des formules de calcul différentes comme cela sera expliqué plus en détail par la suite.

**7.5. Exemple d'un deuxième mode de réalisation procédé selon l'invention**

Ce deuxième mode de réalisation diffère du premier mode de réalisation en ce qu'il comprend une étape de mesure de la pression artérielle en phase systolique (PAS), une étape de mesure de la pression artérielle en phase diastolique (PAD) et une étape de calcul de la pression artérielle moyenne (PAM).

Il en diffère également en ce que, l'ordinateur 6 permet, en fonction des signaux délivrés par l'impédancemètre 1 :

- de déterminer (421) un premier indice intermédiaire (RP) représentatif de la résistance locale du segment artériel au cours d'une phase systolique d'un battement cardiaque ;
- de déterminer (422) un deuxième indice intermédiaire (ID) représentatif de la distensibilité de l'artère.

Plus précisément, l'ordinateur 6 traite trois signaux en vue de déterminer les indices (Ira), (RP) et (ID) :

- le signal d'électrocardiogramme (ECG) du sujet (figure 3a) ;
- le signal représentant l'inverse de la variation d'impédance dans le volume (V) de sang circulant dans la portion d'artère située entre les couples d'électrodes (figure 3b) ;
- le signal représentant la dérivée de l'inverse de la variation d'impédance (figure 3c).

La variation d'impédance ou la dérivée de l'inverse de la variation d'impédance du sang circulant dans le segment d'une artère peut être assimilée à la mesure de l'énergie cinétique circulant à l'intérieur de l'artère de conduction étudiée.

Durant le début de la phase systolique, le sang circulant dans le segment possède une première énergie cinétique EC1 et l'artère emmagasine une énergie mécanique EM1. Pendant la fin de la phase systolique, le sang circulant dans le segment possède une deuxième énergie cinétique EC2 et l'artère emmagasine une énergie mécanique EM2. Durant la phase diastolique, l'artère restitue une énergie mécanique totale égale à la somme des énergies mécaniques EM1 et EM2.

Le premier indice intermédiaire (RP) s'écrit :

$$RP = \frac{PAM}{(EC1 + EC2)}$$

La première énergie cinétique EC1 est égale à I. La deuxième énergie cinétique EC2 est égale à J. On en déduit donc que :

$$RP = \frac{PAM}{(I + J)}$$

Les moyens de calculs 6 déterminent le premier indice intermédiaire (RP) selon la formule :

$$RP = \frac{PAM}{(I + J)}$$

$$\text{avec } I = \int_{t_1}^{t_2} \left| \frac{d}{dt}\left(\frac{1}{\Delta Z}\right) \right| dt \text{ et } J = \int_{t_2}^{t_3} \left| \frac{d}{dt}\left(\frac{1}{\Delta Z}\right) \right| dt,$$

$t_1$ représentant le temps d'apparition du pied de la dérivée de la variation d'impédance $(\frac{d}{dt}\frac{1}{\Delta Z})$ depuis le début de ladite phase systolique,

$t_2$ représentant le temps d'apparition du maximum de la dérivée de la variation d'impédance $(\frac{d}{dt}\frac{1}{\Delta Z})$ depuis le début de ladite phase systolique,

$t_3$ représentant le temps d'apparition de l'intersection de la dérivée de la variation d'impédance $(\frac{d}{dt}\frac{1}{\Delta Z})$ et d'une droite parallèle à l'axe des abscisses passant par le point de la courbe $(\frac{d}{dt}\frac{1}{\Delta Z})$ au temps $t_1$.

Le deuxième indice intermédiaire (ID) s'écrit :

$$ID = \frac{PAM}{(EM1 + EM2)} = PAM\frac{100\left[2(J - I) - (J + I)\right]}{(J + I)^2}$$

Les moyens de calculs 6 déterminent le deuxième indice intermédiaire (ID) selon la formule :

$$ID = PAM\frac{100\left[2(J - I) - (J + I)\right]}{(J + I)^2}$$

Les moyens de calculs 6 déterminent enfin l'indice (Ira) en fonction des premier (RP) et deuxième (ID) indices intermédiaires calculés précédemment selon la formule :

$$Ira = \frac{PAS - PAD}{PAM} \cdot \frac{RP \cdot ID}{RP + ID}$$

L'indice de la rigidité locale de la paroi aortique est fourni par l'indice (Ira) et l'importance relative de la composante résistive et de la composante capacitive responsables de la rigidité artérielle pourra être quantifiée et appréciée de l'homme de métier par l'interprétation des indices intermédiaires (RP) et (ID).

Il a ainsi été constaté expérimentalement, que les valeurs des indices intermédiaires (RP) et (ID) pourront être répertoriés en différentes classes, mentionnées ici à titre purement indicatif, illustrant l'état des artères :

- (RP) inférieur à 5 et (ID) supérieur à 20 indiquent une résistance locale basse et une distensibilité aortique élevée.
- (RP) inférieur à 5 et (ID) inférieur à 5 indiquent une résistance locale basse et une distensibilité aortique basse.
- (RP) supérieur à 20 et (ID) inférieur à 5 indiquent une résistance locale élevée et une distensibilité aortique basse.
- (RP) supérieur à 20 et (ID) supérieur à 20 indiquent une résistance locale élevée et une distensibilité aortique élevée.

## Revendications

1. Procédé de mesure d'un indice (Ira) de la rigidité locale de la paroi d'une artère de conduction véhiculant le sang d'un sujet, **caractérisé en ce que** ledit procédé comprend au moins :

   - une étape de mesure, en un unique point de mesure, de la variation d'impédance électrique ($\Delta Z$) d'un volume (V) du sang circulant dans un segment de ladite artère ;
   - une étape de détermination d'un premier indice intermédiaire ($RP_\%$, RP) représentatif d'une caractéristique résistive mise en jeu dans la rigidification de ladite paroi, et d'un deuxième indice intermédiaire ($PCPA_\%$, ID) représentatif d'une caractéristique capacitive mise en jeu dans la rigidification de ladite paroi, lesdits premier ($RP_\%$, RP) et deuxième ($PCPA_\%$, ID) indices intermédiaires étant obtenus à partir de ladite mesure de la variation d'impédance électrique ($\Delta Z$) ;
   - une étape de détermination dudit indice (Ira) de la rigidité locale en fonction desdits premier ($RP_\%$, RP) et deuxième ($PCPA_\%$, ID) indices intermédiaires.

2. Procédé selon la revendication 1, **caractérisé en ce que** ledit premier indice intermédiaire ($RP_\%$) est un indice représentatif de la résistance périphérique en aval dudit segment au cours d'une phase systolique d'un battement cardiaque, et **en ce que** ledit deuxième indice intermédiaire ($PCPA_\%$) est indice représentatif de la capacité de ladite artère à emmagasiner une énergie mécanique due à la déformation de ladite artère durant ladite phase systolique dudit battement cardiaque et à la restituer durant la phase diastolique dudit battement cardiaque.

3. Procédé selon la revendication 2, **caractérisé en ce que** ladite étape de détermination dudit indice (Ira) de la rigidité locale comprend une étape de calcul selon la formule :

$$Ira = (1 - |PCPA_\%|) \times RP_\% + (1 - RP_\%) * |PCPA_\%|$$

4. Procédé selon la revendication 3, **caractérisé en ce qu'**il comprend une étape de calcul dudit deuxième indice intermédiaire ($PCPA_\%$) selon la formule :

$$PCPA_\% = \frac{J - I}{J + I} * 100$$

$$\text{avec } I = \int_{t_1}^{t_2} \left| \frac{d}{dt}\left(\frac{1}{\Delta Z}\right) \right| dt \text{ et } J = \int_{t_2}^{t_3} \left| \frac{d}{dt}\left(\frac{1}{\Delta Z}\right) \right| dt,$$

$t_1$ représentant le temps d'apparition du pied de la dérivée de l'inverse de la variation d'impédance $\left(\frac{d}{dt}\frac{1}{\Delta Z}\right)$ depuis le début de ladite phase systolique,

$t_2$ représentant le temps d'apparition du maximum de la dérivée de l'inverse de la variation d'impédance

$(\dfrac{d}{dt}\dfrac{1}{\Delta Z})$ depuis le début de ladite phase systolique,

$t_3$ représentant le temps d'apparition de l'intersection de la dérivée de l'inverse de la variation d'impédance

$(\dfrac{d}{dt}\dfrac{1}{\Delta Z})$ et d'une droite parallèle à l'axe des abscisses passant par le point de la courbe $(\dfrac{d}{dt}\dfrac{1}{\Delta Z})$ au temps $t_1$.

5. Procédé selon la revendication 4, **caractérisé en ce qu'**il comprend une étape de calcul dudit premier indice intermédiaire ($RP_\%$) selon la formule :

$$RP_\% = \frac{K-I}{K} * 100$$

K étant une constante dépendant des moyens mis en oeuvre pour réaliser ladite étape de mesure de la variation d'impédance électrique ($\Delta Z$).

6. Procédé selon la revendication 1, **caractérisé en ce que** ledit premier indice intermédiaire est un indice (RP) représentatif de la résistance locale dudit segment au cours d'une phase systolique d'un battement cardiaque, et **en ce que** ledit deuxième indice intermédiaire est indice (ID) représentatif de la distensibilité de ladite artère au cours d'une phase systolique d'un battement cardiaque.

7. Procédé selon la revendication 6, **caractérisé en ce qu'**il comprend une étape de mesure de la pression artérielle en phase systolique (PAS), de la pression artérielle en phase diastolique (PAD) et de calcul de la pression artérielle moyenne (PAM).

8. Procédé selon la revendication 7, **caractérisé en ce que** ladite étape de détermination dudit indice (Ira) de la rigidité locale comprend une étape de calcul selon la formule :

$$Ira = \frac{PAS - PAD}{PAM} \cdot \frac{RP \cdot ID}{RP + ID}$$

9. Procédé selon la revendication 8, **caractérisé en ce qu'**il comprend une étape de calcul dudit premier indice intermédiaire (RP) selon la formule :

$$RP = \frac{PAM}{(I+J)}$$

$$\text{avec } I = \int_{t_1}^{t_2}\left|\frac{d}{dt}\left(\frac{1}{\Delta Z}\right)\right|dt \text{ et } J = \int_{t_2}^{t_3}\left|\frac{d}{dt}\left(\frac{1}{\Delta Z}\right)\right|dt,$$

$t_1$ représentant le temps d'apparition du pied de la dérivée de l'inverse de la variation d'impédance $(\dfrac{d}{dt}\dfrac{1}{\Delta Z})$ depuis le début de ladite phase systolique,

$t_2$ représentant le temps d'apparition du maximum de la dérivée de l'inverse de la variation d'impédance

$(\dfrac{d}{dt}\dfrac{1}{\Delta Z})$ depuis le début de ladite phase systolique,

$t_3$ représentant le temps d'apparition de l'intersection de la dérivée de l'inverse de la variation d'impédance

$(\dfrac{d}{dt}\dfrac{1}{\Delta Z})$ et d'une droite parallèle à l'axe des abscisses passant par le point de la courbe $(\dfrac{d}{dt}\dfrac{1}{\Delta Z})$ au temps $t_1$.

**10.** Procédé selon la revendication 11, **caractérisé en ce qu'**il comprend une étape de calcul dudit deuxième indice intermédiaire (ID) selon la formule

$$ID = PAM \frac{100\big[2(J-I)-(J+I)\big]}{(J+I)^2}$$

**11.** Procédé selon l'une quelconque des revendications 1 à 10, **caractérisé en ce qu'**il comprend une étape d'acquisition du signal électrocardiogramme (ECG) dudit sujet, et une étape de synchronisation dudit signal électrocardiogramme (ECG) et de ladite variation d'impédance ($\Delta Z$).

**12.** Procédé selon l'une quelconque des revendications 1 à 11, **caractérisé en ce qu'**il comprend plusieurs :

- étapes de détermination dudit premier ($RP_\%$, RP) et dudit deuxième ($PCPA_\%$, ID) indices intermédiaires ;
- étapes de détermination dudit indice (Ira) de la rigidité locale en fonction desdits premier ($RP_\%$, RP) et deuxième ($PCPA_\%$, ID) indices intermédiaires,

lesdites étapes de détermination étant menées au cours de battements cardiaques (R) successifs, ledit procédé comprenant de plus une étape de calcul de la moyenne de chacun desdits indices (Ira, $RP_\%$, RP, $PCPA_\%$, ID) au cours desdits battements (R).

**13.** Procédé selon l'une quelconque des revendications 1 à 12, **caractérisé en ce qu'**il comprend plusieurs étapes de mesure, en un unique point de mesure, de la variation d'impédance électrique ($\Delta Z$) d'un volume (V) du sang circulant dans un segment de ladite artère, chacune desdites mesures étant réalisées sur des battements cardiaques (R) différents, ledit procédé comprenant de plus une étape de détermination de la moyenne de la variation d'impédance sur lesdits battement cardiaques et une étape de détermination dudit premier ($RP_\%$, RP) et dudit deuxième ($PCPA_\%$, ID) indices intermédiaires en fonction de ladite moyenne.

**14.** Procédé selon l'une quelconque des revendications 1 à 13, **caractérisé en ce qu'**il comprend une étape d'affichage dudit indice (Ira) de la rigidité locale de la paroi d'une artère de conduction, et une étape d'affichage desdits premier ($RP_\%$, RP) et deuxième ($PCPA_\%$, ID) indices intermédiaires.

**15.** Installation pour la mise en oeuvre du procédé de détermination d'un indice (Ira) de la rigidité locale de la paroi d'une artère de conduction d'un sujet véhiculant du sang selon l'une quelconque des revendications 1 à 14, **caractérisé en ce qu'**elle comprend :

- des moyens de mesure, en un unique point de mesure, de la variation d'impédance électrique ($\Delta Z$) d'un volume (V) du sang circulant dans un segment ladite artère ;
- des moyens de détermination d'un premier indice intermédiaire ($RP_\%$, RP) représentatif d'une caractéristique résistive mise en jeu dans la rigidification de ladite paroi, et d'un deuxième indice intermédiaire ($PCPA_\%$, ID) représentatif d'une caractéristique capacitive mise en jeu dans la rigidification de ladite paroi ;
- des moyens de détermination dudit indice (Ira) de la rigidité locale en fonction desdits premier ($RP_\%$, RP) et deuxième ($PCPA_\%$, ID) indices intermédiaires.

**16.** Installation selon la revendication 15, **caractérisé en ce que** lesdits moyens de détermination dudit premier indice intermédiaire ($RP_\%$) comprennent des moyens de détermination d'un indice représentatif de la résistance périphérique en aval dudit segment au cours d'une phase systolique d'un battement cardiaque, et **en ce que** lesdits moyens de détermination dudit deuxième indice intermédiaire ($PCPA_\%$) comprennent des moyens de détermination d'un indice représentatif de la capacité de ladite artère à emmagasiner une énergie mécanique due à la déformation de ladite artère durant ladite phase systolique dudit battement cardiaque et à la restituer durant la phase diastolique dudit battement cardiaque.

**17.** Installation selon la revendication 16, **caractérisée en ce qu'**elle comprend des moyens de calculs dudit indice (Ira) de la rigidité locale selon la formule :

$$Ira = (1 - |PCPA_\%|) \times RP_\% + (1 - RP_\%) * |PCPA_\%|$$

**18.** Installation selon la revendication 17, **caractérisée en ce que** lesdits moyens de calcul dudit deuxième indice intermédiaire (PCPA%) comprennent des moyens de calcul selon la formule :

$$PCPA_\% = \frac{J - I}{J + I} * 100$$

$$\text{avec } I = \int_{t_1}^{t_2} \left| \frac{d}{dt}\left(\frac{1}{\Delta Z}\right) \right| dt \text{ et } J = \int_{t_2}^{t_3} \left| \frac{d}{dt}\left(\frac{1}{\Delta Z}\right) \right| dt,$$

$t_1$ représentant le temps d'apparition du pied de la dérivée de l'inverse de la variation d'impédance $(\frac{d}{dt}\frac{1}{\Delta Z})$ depuis le début de ladite phase systolique,

$t_2$ représentant le temps d'apparition du maximum de la dérivée de l'inverse de la variation d'impédance $(\frac{d}{dt}\frac{1}{\Delta Z})$ depuis le début de ladite phase systolique,

$t_3$ représentant le temps d'apparition de l'intersection de la dérivée de l'inverse de la variation d'impédance $(\frac{d}{dt}\frac{1}{\Delta Z})$ et d'une droite parallèle à l'axe des abscisses passant par le point de la courbe $(\frac{d}{dt}\frac{1}{\Delta Z})$ au temps $t_1$.

**19.** Installation selon la revendication 18, **caractérisée en ce que** lesdits moyens de calcul dudit premier indice intermédiaire (RP%) comprennent des moyens de calcul selon la formule :

$$RP_\% = \frac{K - I}{K} * 100$$

K étant une constante dépendant des moyens mis en oeuvre pour réaliser ladite étape de mesure de la variation d'impédance électrique (ΔZ).

**20.** Installation selon la revendication 15, **caractérisé en ce que** lesdits moyens de détermination dudit premier indice intermédiaire (RP) comprennent des moyens de détermination d'un indice représentatif de la résistance locale dudit segment au cours d'une phase systolique d'un battement cardiaque, et **en ce que** lesdits moyens de détermination dudit deuxième indice intermédiaire (ID) comprennent des moyens de détermination d'un indice représentatif de la distensibilité au cours d'une phase systolique d'un battement cardiaque.

**21.** Installation selon la revendication 20, **caractérisée en ce qu'**elle comprend des moyens de calcul dudit indice (Ira) de la rigidité locale selon la formule :

$$Ira = \frac{PAS - PAD}{PAM} \cdot \frac{RP \cdot ID}{RP + ID}$$

**22.** Installation selon la revendication 21, **caractérisée en ce que** lesdits moyens de calcul dudit premier indice intermédiaire (RP) comprennent des moyens de calcul selon la formule :

$$RP = \frac{PAM}{(I + J)}$$

$$\text{avec } I = \int_{t_1}^{t_2} \left| \frac{d}{dt}\left(\frac{1}{\Delta Z}\right) \right| dt \text{ et } J = \int_{t_2}^{t_3} \left| \frac{d}{dt}\left(\frac{1}{\Delta Z}\right) \right| dt,$$

$t_1$ représentant le temps d'apparition du pied de la dérivée de l'inverse de la variation d'impédance $(\dfrac{d}{dt}\dfrac{1}{\Delta Z})$ depuis le début de ladite phase systolique,

$t_2$ représentant le temps d'apparition du maximum de la dérivée de l'inverse de la variation d'impédance $(\dfrac{d}{dt}\dfrac{1}{\Delta Z})$ depuis le début de ladite phase systolique,

$t_3$ représentant le temps d'apparition de l'intersection de la dérivée de l'inverse de la variation d'impédance $(\dfrac{d}{dt}\dfrac{1}{\Delta Z})$ et d'une droite parallèle à l'axe des abscisses passant par le point de la courbe $(\dfrac{d}{dt}\dfrac{1}{\Delta Z})$ au temps $t_1$.

23. Installation selon la revendications 22, **caractérisée en ce que** lesdits moyens de calcul dudit deuxième indice intermédiaire (ID) comprennent des moyens de calcul selon la formule :

$$ID = PAM\,\frac{100\big[2(J-I)-(J+I)\big]}{(J+I)^2}$$

24. Installation selon l'une quelconque des revendications 15 à 23, **caractérisée en ce qu'**elle comprend des moyens d'acquisition du signal d'électrocardiogramme (ECG) dudit sujet, des moyens de détection de chacun des battements cardiaques (R) apparaissant sur ledit signal électrocardiogramme (ECG), et des moyens d'activation desdits moyens de détermination desdits indices consécutivement à la détection d'au moins un battement cardiaque (R).

**Patentansprüche**

1. Verfahren zum Messen eines Index (Ira) der lokalen Steifigkeit der Wand einer leitenden Arterie, die Blut eines Subjekts transportiert, **dadurch gekennzeichnet, dass** das Verfahren wenigstens aufweist:

   - einen Schritt des Messens, in einem einzigen Messpunkt, der Variation der elektrischen Impedanz ($\Delta Z$) eines Volumens (V) des in einem Segment der Arterie zirkulierenden Blutes;
   - einen Schritt des Bestimmens eines ersten Zwischenindex ($RP_\%$, RP), der für eine bei der Versteifung der Wand involvierte Widerstandscharakteristik repräsentativ ist, und eines zweiten Zwischenindex ($PCPA_\%$, ID), der für eine bei der Versteifung der Wand involvierte Kapazitätscharakteristik repräsentativ ist, wobei der erste ($RP_\%$, RP) und zweite ($PCPA_\%$, ID) Zwischenindex ausgehend von der Messung der Variation der elektrischen Impedanz ($\Delta Z$) erhalten werden;
   - einen Schritt des Bestimmens des Index (Ira) der lokalen Steifigkeit als Funktion des ersten ($RP_\%$, RP) und des zweiten ($PCPA_\%$, ID) Zwischenindex.

2. Verfahren nach Anspruch 1, **dadurch gekennzeichnet, dass** der erste Zwischenindex ($RP_\%$) ein Index ist, der für den peripheren Widerstand nachgelagerten des Segments während einer systolischen Phase eines Herzschlages repräsentativ ist, und dadurch, dass der zweiten Zwischenindex ($PCPA_\%$) ein Index ist, der für die Kapazität der Arterie zum Speichern einer mechanischen Energie aufgrund einer Verformung der Arterie während der systolischen Phase des Herzschlages und zum Freigeben selbiger während der diastolischen Phase des Herzschlages repräsentativ ist.

3. Verfahren nach Anspruch 1, **dadurch gekennzeichnet, dass** der Schritt des Bestimmens des Index (Ira) der lokalen Steifigkeit einen Schritt des Berechnens nach der Formel:

$$Ira = (1-\big|PCPA_\%\big|)\,x\,RP_\% + (1-RP_\%)*\big|PCPA_\%\big|$$

aufweist.

4. Verfahren nach Anspruch 3, **dadurch gekennzeichnet, dass** es einen Schritt des Berechnens des zweiten Zwischenindex (PCPA$_\%$) gemäß der Formel:

$$PCPA_{\%} = \frac{J-1}{J+1} * 100$$

aufweist, mit $I = \int\limits_{t1}^{t2} \left| \frac{d}{dt}\left( \frac{1}{\Delta Z} \right) \right| dt$   und   $J = \int\limits_{t2}^{t3} \left| \frac{d}{dt}\left( \frac{1}{\Delta Z} \right) \right| dt$,

$t_1$ repräsentiert den Zeitpunkt des Auftretens der Basis der Ableitung des Inversen der Variation der Impedanz $\left( \frac{d}{dt}\frac{1}{\Delta Z} \right)$ nach dem Beginn der systolischen Phase,

$t_2$ repräsentiert den Zeitpunkt des Auftretens des Maximums der Ableitung des Inversen der Variation der Impedanz $\left( \frac{d}{dt}\frac{1}{\Delta Z} \right)$ nach dem Beginn der systolischen Phase,

$t_3$ repräsentiert den Zeitpunkt des Auftretens des Schnittpunktes der Ableitung des Inversen der Variation der Impedanz $\left( \frac{d}{dt}\frac{1}{\Delta Z} \right)$ und einer Geraden, die parallel zu der Abszissenachse ist und durch den Punkt der Kurve $\left( \frac{d}{dt}\frac{1}{\Delta Z} \right)$ zum Zeitpunkt $t_1$ verläuft.

**5.** Verfahren nach Anspruch 4, **dadurch gekennzeichnet, dass** es einen Schritt des Berechnens des ersten Zwischenindex (RP$_\%$) gemäß der Formel:

$$RP_{\%} = \frac{K-1}{K} * 100$$

umfasst, wobei K eine Konstante ist, die von den zum Realisieren des Schrittes des Messens der Variation der elektrischen Impedanz ($\Delta Z$) eingesetzten Mitteln abhängt.

**6.** Verfahren nach Anspruch 1, **dadurch gekennzeichnet, dass** der erste Zwischenindex ein Index (RP) ist, der für den lokalen Widerstand des Segments während einer systolischen Phase eines Herzschlags repräsentativ ist, und dadurch, dass der zweite Zwischenindex ein Index (ID) ist, der für die Ausdehnungsfähigkeit (distensibility auf English) der Arterie während einer systolischen Phase eines Herzschlags repräsentativ ist.

**7.** Verfahren nach Anspruch 6, **dadurch gekennzeichnet, dass** es einen Schritt des Messens des Arteriendruckes in systolischer Phase (PAS), des Arteriendruckes in diastolischer Phase (PAD) und des Berechnens des mittleren Arteriendruckes (PAM) aufweist.

**8.** Verfahren nach Anspruch 7, **dadurch gekennzeichnet, dass** der Schritt des Bestimmens des Index (Ira) der lokalen Steifigkeit einen Schritt des Berechnens gemäß der Formel:

$$Ira = \frac{PAS - PAD}{PAM} \cdot \frac{RP \cdot ID}{RP + ID}$$

aufweist.

**9.** Verfahren nach Anspruch 8, **dadurch gekennzeichnet, dass** es ein Schritt des Berechnens des ersten Zwischenindex (RP) gemäß der Formel:

$$RP = \frac{PAM}{(I+J)}$$

aufweist, mit $I = \int_{t1}^{t2} \left| \frac{d}{dt}\left( \frac{1}{\Delta Z} \right) \right| dt$   und   $J = \int_{t2}^{t3} \left| \frac{d}{dt}\left( \frac{1}{\Delta Z} \right) \right| dt,$

$t_1$ repräsentiert den Zeitpunkt des Auftretens der Basis der Ableitung des Inversen der Variation der Impedanz $\left( \frac{d}{dt}\frac{1}{\Delta Z} \right)$ nach dem Beginn der systolischen Phase,

$t_2$ repräsentiert den Zeitpunkt des Auftretens des Maximums der Ableitung des Inversen der Variation der Impedanz $\left( \frac{d}{dt}\frac{1}{\Delta Z} \right)$ nach dem Beginn der systolischen Phase,

$t_3$ repräsentiert den Zeitpunkt des Auftretens des Schnittpunktes der Ableitung des Inversen der Variation der Impedanz $\left( \frac{d}{dt}\frac{1}{\Delta Z} \right)$ und einer Geraden, die parallel zu der Abszissenachse ist und durch den Punkt der Kurve $\left( \frac{d}{dt}\frac{1}{\Delta Z} \right)$ zum Zeitpunkt $t_1$ verläuft.

10. Verfahren nach Anspruch 11, **dadurch gekennzeichnet, dass** es einen Schritt des Berechnens des zweiten Zwischenindex (ID) gemäß der Formel

$$ID = PAM \frac{100\left[ 2(J-I)-(J+I) \right]}{(J+I)^2}$$

aufweist.

11. Verfahren nach einem der Ansprüche 1 bis 10, **dadurch gekennzeichnet, dass** es einen Schritt des Erlangens eines Elektrokardiogrammsignals (ECG) des Subjekts und einen Schritt des Synchronisierens des Elektrokardiogrammsignals (ECG) und der Variation der Impedanz ($\Delta Z$) aufweist.

12. Verfahren nach einem der Ansprüche 1 bis 11, **dadurch gekennzeichnet, dass** es mehrfach aufweist:

- Schritte des Bestimmens des ersten ($RP_\%$, RP) und des zweiten ($PCPA_\%$, ID) Zwischenindex;
- Schritte des Bestimmens des Index (Ira) der lokalen Steifigkeit als Funktion des ersten ($RP_\%$, RP) und des zweiten ($PCPA_\%$, ID) Zwischenindex,
wobei die Schritte des Bestimmens während aufeinanderfolgenden Herzschlägen (R) durchgeführt werden,
wobei das Verfahren zusätzlich einen Schritt des Berechnens des Mittelwertes jedes der Indizes (Ira, $RP_\%$, RP, $PCPA_\%$, ID) über die Schläge (R) aufweist.

13. Verfahren nach einem der Ansprüche 1 bis 12, **dadurch gekennzeichnet, dass** es mehrere Schritte des Messens, in einem einzigen Messpunkt, der Variation der elektrischen Impedanz ($\Delta Z$) eines Volumens (V) des in einem Segment der Arterie zirkulierenden Blutes umfasst, wobei jede der Messungen auf verschiedenen Herzschlägen (R) realisiert wird, wobei das Verfahren zusätzlich einen Schritt des Bestimmens des Mittelwertes der Variation der Impedanz über die Herzschläge und einen Schritt des Bestimmens des ersten ($RP_\%$, RP) und des zweiten ($PCPA_\%$, ID) Zwischenindex als Funktion des Mittelwertes aufweist.

14. Verfahren nach einem der Ansprüche 1 bis 13, **dadurch gekennzeichnet, dass** es einen Schritt des Anzeigens des Index (Ira) der lokalen Steifigkeit der Wand einer leitenden Arterie und einen Schritt des Anzeigens des ersten (RP%, RP) und des zweiten (PCPA%, ID) Zwischenindex aufweist.

15. Einrichtung zum Ausführen des Verfahrens zum Bestimmen eines Index (Ira) der lokalen Steifigkeit einer leitenden Arterie, die Blut eines Subjekts transportiert, nach einem der Ansprüche 1 bis 14, **dadurch gekennzeichnet, dass** sie aufweist:

- ein Mittel zum Messen, in einem einzigen Messpunkt, der Variation der elektrischen Impedanz (ΔZ) eines Volumens (V) des in einem Segment der Arterie zirkulierenden Blutes;
- ein Mittel zum Bestimmen eines ersten Zwischenindex (RP%, RP), der für eine bei der Versteifung der Wand involvierte Widerstandscharakteristik repräsentativ ist, und eines zweiten Zwischenindex (PCPA%, ID), der für eine bei der Versteifung der Wand involvierte Kapazitätscharakteristik repräsentativ ist, wobei der erste (RP%, RP) und zweite (PCPA%, ID) Zwischenindex ausgehend von der Messung der Variation der elektrischen Impedanz (ΔZ) erhalten werden;
- ein Mittel zum Bestimmen des Index (Ira) der lokalen Steifigkeit als Funktion des ersten (RP%, RP) und des zweiten (PCPA%, ID) Zwischenindex.

16. Einrichtung nach Anspruch 15, **dadurch gekennzeichnet, dass** das Mittel zum Bestimmen des ersten Zwischenindex (RP%) ein Mittel zum Bestimmen eines Index aufweist, der für den peripheren Widerstand nachgelagerten des Segments während einer systolischen Phase eines Herzschlages repräsentativ ist, und dadurch, dass das Mittel zum Bestimmen des zweiten Zwischenindex (PCPA%) ein Mittel zum Bestimmen eines Index aufweist, der für die Kapazität der Arterie zum Speichern einer mechanischen Energie aufgrund einer Verformung der Arterie während der systolischen Phase des Herzschlages und zum Freigeben selbiger während der diastolischen Phase des Herzschlages repräsentativ ist.

17. Einrichtung nach Anspruch 16, **dadurch gekennzeichnet, dass** sie ein Mittel zum Berechnen des Index (Ira) der lokalen Steifigkeit gemäß der folgenden Formel aufweist:

$$Ira = (1 - |PCPA_\%|) \, x \, RP_\% + (1 - RP_\%) * |PCPA_\%|$$

18. Einrichtung nach Anspruch 17, **dadurch gekennzeichnet, dass** das Mittel zum Berechnen des zweiten Zwischenindex (PCPA%) ein Mittel zum Berechnen gemäß der Formel:

$$PCPA_\% = \frac{J-1}{J+1} * 100$$

aufweist, mit $I = \int_{t1}^{t2} \left| \frac{d}{dt}\left(\frac{1}{\Delta Z}\right) \right| dt$ und $J = \int_{t2}^{t3} \left| \frac{d}{dt}\left(\frac{1}{\Delta Z}\right) \right| dt$,

$t_1$ repräsentiert den Zeitpunkt des Auftretens der Basis der Ableitung des Inversen der Variation der Impedanz $\left(\frac{d}{dt}\frac{1}{\Delta Z}\right)$ nach dem Beginn der systolischen Phase,

$t_2$ repräsentiert den Zeitpunkt des Auftretens des Maximums der Ableitung des Inversen der Variation der Impedanz $\left(\frac{d}{dt}\frac{1}{\Delta Z}\right)$ nach dem Beginn der systolischen Phase,

$t_3$ repräsentiert den Zeitpunkt des Auftretens des Schnittpunktes der Ableitung des Inversen der Variation der Impedanz $\left(\frac{d}{dt}\frac{1}{\Delta Z}\right)$ und einer Geraden, die parallel zu der Abszissenachse ist und durch den Punkt der

Kurve $\left( \dfrac{d}{dt} \dfrac{1}{\Delta Z} \right)$ zum Zeitpunkt $t_1$ verläuft.

**19.** Einrichtung nach Anspruch 18, **dadurch gekennzeichnet, dass** das Mittel zum Berechnen des ersten Zwischenindex (RP$_\%$) ein Mittel zum Berechnen gemäß der Formel:

$$RP_\% = \frac{K-1}{K} * 100$$

aufweist, wobei K eine Konstante ist, die von dem zum Realisieren des Schrittes des Messens der Variation der elektrischen Impedanz ($\Delta Z$) eingesetzten Mittel abhängt.

**20.** Einrichtung nach Anspruch 15, **dadurch gekennzeichnet, dass** das Mittel zum Bestimmen des ersten Zwischenindex (RP) ein Mittel zum Bestimmen eines Index aufweisen, der für den lokalen Widerstand des Segments während einer systolischen Phase eines Herzschlags repräsentativ ist, und dadurch, dass das Mittel zum Bestimmen des zweiten Zwischenindex (ID) ein Mittel zum Bestimmen eines Index aufweisen, der für die Ausdehnungsfähigkeit (distensibility auf English) während einer systolischen Phase eines Herzschlags repräsentativ ist.

**21.** Einrichtung nach Anspruch 20, **dadurch gekennzeichnet, dass** sie ein Mittel zum Berechnen des Index (Ira) der lokalen Steifigkeit gemäß der folgenden Formel aufweist:

$$Ira = \frac{PAS - PAD}{PAM} \cdot \frac{RP \cdot ID}{RP + ID}$$

**22.** Einrichtung nach Anspruch 21, **dadurch gekennzeichnet, dass** das Mittel zum Berechnen des ersten Zwischenindex (RP) ein Mittel zum Berechnen gemäß der Formel:

$$RP = \frac{PAM}{(I+J)}$$

aufweist, mit $I = \int\limits_{t1}^{t2} \left| \dfrac{d}{dt} \left( \dfrac{1}{\Delta Z} \right) \right| dt$ und $J = \int\limits_{t2}^{t3} \left| \dfrac{d}{dt} \left( \dfrac{1}{\Delta Z} \right) \right| dt$,

$t_1$ repräsentiert den Zeitpunkt des Auftretens der Basis der Ableitung des Inversen der Variation der Impedanz $\left( \dfrac{d}{dt} \dfrac{1}{\Delta Z} \right)$ nach dem Beginn der systolischen Phase,

$t_2$ repräsentiert den Zeitpunkt des Auftretens des Maximums der Ableitung des Inversen der Variation der Impedanz $\left( \dfrac{d}{dt} \dfrac{1}{\Delta Z} \right)$ nach dem Beginn der systolischen Phase,

$t_3$ repräsentiert den Zeitpunkt des Auftretens des Schnittpunktes der Ableitung des Inversen der Variation der Impedanz $\left( \dfrac{d}{dt} \dfrac{1}{\Delta Z} \right)$ und einer Geraden, die parallel zu der Abszissenachse ist und durch den Punkt der Kurve $\left( \dfrac{d}{dt} \dfrac{1}{\Delta Z} \right)$ zum Zeitpunkt $t_1$ verläuft.

**23.** Einrichtung nach Anspruch 22, **dadurch gekennzeichnet, dass** das Mittel zum Berechnen des zweiten Zwischenindex (ID) ein Mittel zum Berechnen gemäß der Formel:

$$ID = PAM \frac{100[2(J-I)-(J+I)]}{(J+I)^2}$$

aufweist.

**24.** Einrichtung nach einem der Ansprüche 15 bis 23, **dadurch gekennzeichnet, dass** sie ein Mittel zum Erlangen eines Elektrokardiogrammsignals (ECG) des Subjekts, ein Mittel zum Erkennen jedes der Herzschläge (R), die in dem Elektrokardiogrammsignal (ECG) erscheinen und ein Mittel zum Aktivieren das Mittels zum Bestimmen der Indizes in der Folge einer Erkennung wenigstens eines Herzschlags (R) aufweist.

**Claims**

**1.** A method of measuring an index (Ira) of local stiffness of the wall of a conducting artery, transmitting the blood of a subject, **characterised in that** the said method includes at least:

- one measuring step, in one single measuring point, of electrical impedance variation ($\Delta Z$) of a volume (V) of blood circulating in a segment of the said artery;
- one step for determining a first intermediary index ($RP_\%$, RP), representative of a resistive characteristic brought into play in the stiffening of the said wall, and a second intermediary index ($PCPA_\%$, ID), representative of a capacitive characteristic brought into play in the stiffening of the said wall, the said first ($RP_\%$, RP) and second ($PCPA_\%$, ID) intermediary indexes being obtained from the said measuring step of electrical impedance variation ($\Delta Z$);
- one step for determining the said index (Ira) of the local stiffness in accordance with the said first ($RP_\%$, RP) and second ($PCPA_\%$, ID) intermediary indexes.

**2.** Method according to Claim 1, **characterised in that** the said first intermediary index ($RP_\%$) is an index representative of the peripheral resistance downstream of the said segment during a systolic phase of a heartbeat, and **in that** the said second intermediary index ($PCPA_\%$) is an index representative of the capacity of the said artery to store a mechanical energy, due to the deformation of the said artery during the said systolic phase of the said heartbeat, and to release it during the diastolic phase of the said heartbeat.

**3.** Method according to Claim 2, **characterised in that** the said step for determining the said index (Ira) of the local stiffness, includes a calculation step, according to the formula:

$$Ira = (1 - |PCPA_\%|) \times RP_\% + (1 - RP_\%) * |PCPA_\%|$$

**4.** Method according to Claim 3, **characterised in that** it includes a step for calculating the said second intermediary index ($PCPA_\%$), according to the formula:

$$PCPA_\% = \frac{J-I}{J+I} * 100$$

with $\quad I = \int_{t_1}^{t_2} \left| \frac{d}{dt}\left(\frac{1}{\Delta Z}\right) \right| dt \quad$ and $\quad J = \int_{t_2}^{t_3} \left| \frac{d}{dt}\left(\frac{1}{\Delta Z}\right) \right| dt,$

$t_1$ representing the time for the base of the derivative of the reverse of the impedance variation $(\frac{d}{dt}\frac{1}{\Delta Z})$ from

23

the start of the said systolic phase to appear,

$t_2$ representing the time for the maximum of the derivative of the reverse of the impedance variation $(\frac{d}{dt}\frac{1}{\Delta Z})$

from the start of the said systolic phase to appear,

$t_3$ representing the time for the intersection of the derivative of the reverse of the impedance variation $(\frac{d}{dt}\frac{1}{\Delta Z})$

and a right parallel to the X-axis passing through the curve point $(\frac{d}{dt}\frac{1}{\Delta Z})$ at time $t_1$ to appear.

**5.** Method according to Claim 4, **characterised in that** it includes a step for calculating the said first intermediary index ($RP_\%$), according to the formula:

$$RP_\% = \frac{K - I}{K} * 100$$

K being a constant, dependent on the means implemented to produce the said measuring step of the electrical impedance variation ($\Delta Z$).

**6.** Method according to Claim 1, **characterised in that** the said first intermediary index is an index (RP) representative of the local resistance of the said segment during a systolic phase of a heartbeat, and **in that** the said second intermediary index is an index (ID), representative of the distensibility of the said artery during the systolic phase of a heartbeat.

**7.** Method according to Claim 6, **characterised in that** it includes a step for measuring the arterial pressure in the systolic phase (PAS), the arterial pressure in the diastolic phase (PAD) and calculating the average arterial pressure (PAM).

**8.** Method according to Claim 7, **characterised in that** the said step for determining the said index (Ira) of local stiffness, includes a calculation step, according to the formula:

$$Ira = \frac{PAS - PAD}{PAM} \cdot \frac{RP \cdot ID}{RP + ID}$$

**9.** Method according to Claim 8, **characterised in that** it includes a step for calculating the said first intermediary index (RP), according to the formula:

$$RP = \frac{PAM}{(I + J)}$$

with $I = \int_{t_1}^{t_2} \left| \frac{d}{dt}\left(\frac{1}{\Delta Z}\right) \right| dt$ and $J = \int_{t_2}^{t_3} \left| \frac{d}{dt}\left(\frac{1}{\Delta Z}\right) \right| dt$,

$t_1$ representing the time for the base of the derivative of the reverse of the impedance variation $(\frac{d}{dt}\frac{1}{\Delta Z})$

from the start of the said systolic phase to appear,

$t_2$ representing the time for the maximum of the derivative of the reverse of the impedance variation $(\frac{d}{dt}\frac{1}{\Delta Z})$

from the start of the said systolic phase to appear,

$t_3$ representing the time for the intersection of the derivative of the reverse of the impedance variation $(\frac{d}{dt}\frac{1}{\Delta Z})$

and a right parallel to the X-axis passing through the curve point $(\frac{d}{dt}\frac{1}{\Delta Z})$ at time $t_1$ to appear.

**10.** Method according to Claim 11, **characterised in that** it includes a step for calculating the said second intermediary index (ID), according to the formula:

$$ID = PAM \frac{100\left[2(J-I)-(J+I)\right]}{(J+I)^2}$$

**11.** Method according to any one of Claims 1 to 10, **characterised in that** it includes a step for acquiring an electrocardiogram (ECG) signal from the said subject, and a step for synchronising the said electrocardiogram (ECG) signal and the said impedance variation ($\Delta Z$).

**12.** Method according to any one of Claims 1 to 11, **characterised in that** it includes several:

- steps for determining the said first ($RP_\%$, RP) and the said second ($PCPA_\%$, ID) intermediary indexes;
- steps for determining the said index (Ira) of the local stiffness, in accordance with the said first ($RP_\%$, RP) and second ($PCPA_\%$, ID) intermediary indexes,

the said steps for determining being led during successive heartbeats (R), the said method including, in addition, one step for calculating the average from each one of the said indexes (Ira, $RP_\%$, RP, $PCPA_\%$, ID) during the said beats (R).

**13.** Method according to any one of Claims 1 to 12, **characterised in that** it includes several steps for measuring, in one single measuring point, the electrical impedance variation ($\Delta Z$) of a volume (V) of the blood circulating in a segment of the said artery, each one of the said measurement being made on different heartbeats (R), the said method including, in addition, one step for determining the average of the impedance variation on the said heartbeats, and a step for determining the said first ($RP_\%$, RP) and the said second ($PCPA_\%$, ID) intermediary indexes, in accordance with the said average.

**14.** Method according to any one of Claims 1 to 13, **characterised in that** it includes a step for displaying the said index (Ira) of the local stiffness of the wall of a conducting artery, and a step for displaying the said first ($RP_\%$, RP) and second ($PCPA_\%$, ID) intermediary indexes.

**15.** Installation for implementing the method for determining an index (Ira) of the local stiffness of the wall of a conducting artery of a subject transmitting blood, according to any one of Claims 1 to 14, **characterised in that** it includes:

- means for measuring, in one single measuring point, the electrical impedance variation ($\Delta Z$) of a volume (V) of the blood circulating in a segment of the said artery;
- means for determining a first intermediary index ($RP_\%$, RP), representative of a resistive characteristic brought into play in the stiffening of the said wall, and a second intermediary index ($PCPA_\%$, ID), representative of a capacitive characteristic brought into play in the stiffening of the said wall;
- means for determining the said index (Ira) of the local stiffness, in accordance with the said first ($RP_\%$, RP) and second ($PCPA_\%$, ID) intermediary indexes.

**16.** Installation according to Claim 15, **characterised in that** the said means for determining the said first intermediary index ($RP_\%$) include means for determining an index representative of the peripheral resistance downstream of the said segment during a systolic phase of a heartbeat, and **in that** the said means for determining the said second intermediary index ($PCPA_\%$) include means for determining an index, representative of the capacity of the said artery to store a mechanical energy due to the deformation of the said artery during the said systolic phase of the said heartbeat, and to release it during the diastolic phase of the said heartbeat.

**17.** Installation according to Claim 16, **characterised in that** it includes means for calculating the said index (Ira) of the local stiffness, according to the formula:

$$Ira = (1 - \left| PCPA_\% \right|) \times RP_\% + (1 - RP_\%) * \left| PCPA_\% \right|$$

**18.** Installation according to Claim 17, **characterised in that** the said means for calculating the said second intermediary index ($PCPA_\%$) include calculation means, according to the formula:

$$PCPA_\% = \frac{J - I}{J + I} * 100$$

$$\text{with} \quad I = \int_{t_1}^{t_2} \left| \frac{d}{dt} \left( \frac{1}{\Delta Z} \right) \right| dt \quad \text{and} \quad J = \int_{t_2}^{t_3} \left| \frac{d}{dt} \left( \frac{1}{\Delta Z} \right) \right| dt,$$

$t_1$ representing the time for the base of the derivative of the reverse of the impedance variation $(\frac{d}{dt} \frac{1}{\Delta Z})$ from the start of the said systolic phase to appear,

$t_2$ representing the time for the maximum of the derivative of the reverse of the impedance variation $(\frac{d}{dt} \frac{1}{\Delta Z})$ from the start of the said systolic phase to appear,

$t_3$ representing the time for the intersection of the derivative of the reverse of the impedance variation $(\frac{d}{dt} \frac{1}{\Delta Z})$

and a right parallel to the X-axis passing through the curve point $(\frac{d}{dt} \frac{1}{\Delta Z})$ at time $t_1$ to appear.

**19.** Installation according to Claim 18, **characterised in that** the said means for calculating the said first intermediary index ($RP_\%$) include calculation means, according to the formula:

$$RP_\% = \frac{K - I}{K} * 100$$

K being a constant, depending on the means implemented to produce the said measuring step of the electrical impedance variation ($\Delta Z$).

**20.** Installation according to Claim 15, **characterised in that** the said means for determining the said first intermediary index (RP) include means for determining an index representative of the local resistance of the said segment, during a systolic phase of a heartbeat, and **in that** the said means for determining the said second intermediary index (ID) include means for determining an index representative of the distensibility during a systolic phase of a heartbeat.

**21.** Installation according to Claim 20, **characterised in that** it includes means for calculating the said index (Ira) of the local stiffness, according to the formula:

$$Ira = \frac{PAS - PAD}{PAM} \cdot \frac{RP \cdot ID}{RP + ID}$$

**22.** Installation according to Claim 21, **characterised in that** the said means for calculating the said first intermediary index (RP) include calculation means, according to the formula:

$$RP = \frac{PAM}{(I + J)}$$

with $\quad I = \int_{t_1}^{t_2} \left| \frac{d}{dt}\left(\frac{1}{\Delta Z}\right) \right| dt \quad$ and $\quad J = \int_{t_2}^{t_3} \left| \frac{d}{dt}\left(\frac{1}{\Delta Z}\right) \right| dt,$

$t_1$ representing the time for the base of the derivative of the reverse of the impedance variation $(\frac{d}{dt}\frac{1}{\Delta Z})$ from the start of the said systolic phase to appear,

$t_2$ representing the time for the maximum of the derivative of the reverse of the impedance variation $(\frac{d}{dt}\frac{1}{\Delta Z})$ from the start of the said systolic phase to appear,

$t_3$ representing the time for the intersection of the derivative of the reverse of the impedance variation $(\frac{d}{dt}\frac{1}{\Delta Z})$

and a right parallel to the X-axis passing through the curve point $(\frac{d}{dt}\frac{1}{\Delta Z})$ at time $t_1$ to appear.

**23.** Installation according to Claim 22, **characterised in that** the said means for calculating the said second intermediary index (ID) include calculation means, according to the formula:

$$ID = PAM \frac{100\left[2(J - I) - (J + I)\right]}{(J + I)^2}$$

**24.** Installation according to any one of Claims 15 to 23, **characterised in that** it includes means for acquiring the electrocardiogram (ECG) signal from the said subject, means for detecting each one of the heartbeats (R) appearing on the said electrocardiogram (ECG) signal, and means for activating the said means for determining the said indexes, following the detection of at least one heartbeat (R).

Fig. 1

Fig. 2

ECG

R    R    R

temps

<u>Fig. 3a</u>

$\dfrac{1}{\Delta Z}$

temps

<u>Fig. 3b</u>

$\dfrac{d}{dt}\dfrac{1}{\Delta Z}$

t1 t3

t2

temps

<u>Fig. 3c</u>

421  41       422   42

Mesure de
$\Delta(Z)$

Détermination d'un
premier indice
intermédiaire
(RP%, RP)

Détermination d'un
deuxième indice
intermédiaire
(PCPA%, ID)

Détermination
d'un indice (Ira)  43

<u>Fig. 4</u>   Affichage des indices
(Ira), (PCPA%, ID), (RP%, RP)  44

## RÉFÉRENCES CITÉES DANS LA DESCRIPTION

**Documents brevets cités dans la description**

- JP 2003169779 B
- FR 2707860 A
- US 4562843 A
- US 6577897 B1

**Littérature non-brevet citée dans la description**

- **LAURENT STEPHANE et al.** Expert consensus document on arterial stiffness: methodological issues and clinical applications. *EUROPEAN HEART JOURNAL,* Novembre 2006, vol. 27 (21), 2588-2605
- IMPEDANCE PLETHYSMOGRAPHY FOR THE EVALUATION OF PULSE-WAVE VELOCITY IN LIMBS. **RISACHER F.** MEDICAL AND BIOLOGICAL ENGINEERING AND COMPUTING. SPRINGER, 01 Mai 1993, vol. 31, 318-322